(19) Europäisches Patentamt

European Patent Office

Office européen des brevets

(11) **EP 1 653 232 A1**

(12) **EUROPEAN PATENT APPLICATION**

(43) Date of publication:
03.05.2006 Bulletin 2006/18

(51) Int Cl.:
*G01N 33/543* (2006.01)

(21) Application number: 04405661.2

(22) Date of filing: 27.10.2004

(84) Designated Contracting States:
**AT BE BG CH CY CZ DE DK EE ES FI FR GB GR
HU IE IT LI LU MC NL PL PT RO SE SI SK TR**
Designated Extension States:
**AL HR LT LV MK**

(71) Applicant: **CSEM
Centre Suisse d'Electronique et de
Microtechnique SA
2007 Neuchâtel (CH)**

(72) Inventors:
• **Auerswald, Janko
6004 Luzern (CH)**
• **Knapp, Helmut
6030 Ebikon (CH)**

(74) Representative: **Schneider Feldmann AG
Patent- und Markenanwälte
Beethovenstrasse 49
Postfach 623
8039 Zürich (CH)**

(54) **Method for quantitative evaluation of bead-based affinity assays**

(57)     The method is useful for quantitative evaluation of bead-based lab-on-a-chip affinity assay fluorescence measurements. Fluorescent beads coated with probe molecules are immobilized (11) on a substrate. The fluorescence of the immobilized beads is measured (12) and represents the number of beads. The beads are then perfused with an analyte solution such that analyte molecules are bound to the beads. The analyte molecules are labeled with a second, different fluorescent marker which is used in a second fluorescence measurement (14) to determine the number of analyte molecules bound to the beads. A calculation (15), e.g., a quotient formation, is performed with the first (12) and the second (14) fluorescence measurement.

Fig. 1

**Description**

FIELD OF THE INVENTION

The present invention relates to a method for quantitative evaluation of bead-based affinity assays, such as lab-on-a-chip affinity-assay fluorescence measurements, according to the preamble of the first claim. The invention is useful in medical diagnostics, biotechnology, drug development, life sciences and other fields.

BACKGROUND OF THE INVENTION

In life-sciences research and medical diagnostics, lab-on-a-chip devices offer advantages such as less consumption of sample and expensive reagents, considerably shorter assay time because of shorter diffusion paths, and controlled handling of micro- and nanoparticles, e.g., cells, beads or molecules. Lab-on-a-chip systems often exploit the lock-and-key principle, i.e., a specific biochemical reaction between probe molecules and the detected molecules (affinity assay). In many applications, the probe molecules have to be immobilized on a chip surface in a complex and expensive procedure.

Bead-based assays can be an interesting alternative to direct probe molecule deposition on the chip surface. Here, the probe molecules are attached to bead surfaces by specialized suppliers in cost-efficient large-scale production. The probe-molecule lifetime in original bead suspensions is usually longer than on a dry chip surface. The beads are introduced into the lab-on-a-chip system upon demand. Bead-based platforms offer a high degree of versatility because many kinds of beads with a variety of functionalizations are available. Different approaches are possible to immobilize beads on a chip surface to obtain a monolayer pattern of beads, e.g., mechanical barriers, magnetic contraptions, or retention by positive dielectrophoresis (DEP). The retention of beads by positive DEP offers several advantages, e.g., simple chip layout with planar electrodes, good compatibility with miniaturization and the option to separate different beads according to their dielectric properties or sizes. However, in fluids with a conductivity of above 100 mS/m positive DEP becomes too weak to effectively retain the beads in a flow-through device. Physiological buffers and body fluids usually have conductivities up to 2000 mS/m.

To overcome this obstacle, a bead immobilization method was developed which uses a combination of a slight pressure-driven flow and, simultaneously, AC electroosmotic flow, positive DEP and adhesion, three very strong effects in the microworld. After fast bead immobilization at the electrodes, the DEP voltage can be turned off and the functionalized beads serve as substrates for the detection of analyte molecules in high-conductivity fluids. The fast on-chip immobilization of beads coated with biotin, protein A and goat anti-mouse IgG was demonstrated with this method. The detection of streptavidin molecules was performed as a demonstration assay based on the new bead immobilization method. The immobilization mechanism is disclosed in WO-2004/024333. An alternative immobilization mechanism is retention in vortices generated by alternative-current electro-osmotic flow (AC EOF) or pressure-driven counterflow, as described in WO-02/092222.

For quantitative affinity assays, it is always necessary to know the number of available binding sites.

SUMMARY OF THE INVENTION

It is therefore an object of this invention to provide a method for quantitative evaluation of bead-based affinity assays. This problem and other problems are solved by the method as defined in the first claim. Advantageous embodiments of the invention are given in the dependent claims.

The method according to the invention comprises the steps of:

immobilizing microparticles on a substrate;
determining a quantity characteristic of the number of microparticles immobilized on a certain area of the substrate;
binding analyte molecules to the microparticles;
determining a second quantity characteristic of the number of analyte molecules bound in said area of the substrate; and
performing a calculation with the first and the second quantity.

In preferred embodiments, the second quantity is selected from the group consisting of a fluorescence signal, a signal generated by colloidal gold labels, a signal generated by enzymatic activity, an electro-chemo-luminescence (ECL) signal and an enzyme-linked immunosorbent assay (ELISA) signal. The ligand assay is preferably a sandwich assay or a competitive assay.

The method according to the invention can be applied for all bead-based affinity assays, where beads coated with probe molecules are immobilized in a two-dimensional pattern, preferably as a closed monolayer or a monolayer pattern, on a chip surface or in certain areas on a chip surface. The immobilization includes the immobilization mechanism described in WO-2004/024333, but also retention of beads by mechanical barriers, magnetic retention, retention by dielectrophoresis alone or other mechanisms, where probe beads are trapped in a certain area on a chip.

In a preferred embodiment, fluorescent beads are provided. The bead material may be intrinsically fluorescent, and/or the beads may be marked extrinsically. The fluorescence of the immobilized beads is measured and represents the number of beads. Alternatively, the number of beads could be determined by other means, e.g., by taking an image of the substrate and applying an appropriate image-recognition method to the image. The number of available binding sites is then calculated from the thus determined number of beads and the number of binding sites per bead, a quantity which is usually known.

After immobilization, the beads are perfused with an analyte solution such that analyte molecules are bound to the bead surfaces. The analyte molecules are labeled with a second, different fluorescent marker which is used in a second fluorescence measurement to determine the number of analyte molecules bound to the bead surfaces. The setup and the fluorescent dyes must be chosen in a way that the fluorescence of the beads can not excite the fluorescence of the analyte-molecule marker.

The calculation performed with the first and the second quantity typically comprises the calculation of a quotient of the second and the first quantity, or vice versa, but may alternatively comprise other and more complex algorithms.

The invention offers, amongst others, the following advantages:

- It makes feasible a simple and quick quantitative evaluation of bead-based lab-on-a-chip fluorescence measurements. Bead-based lab-on-a-chip assays become more and more popular because beads are easier to handle in microfluidic systems than molecules.
- Chip surface functionalization with probe molecules carrying beads can be done upon demand shortly before the assay. Probe-molecule shelf life in original bead suspension is longer than on a dry chip surface.
- The use of the method according to the invention in combination with on-chip bead-based affinity assay can be applied for various mechanisms of bead immobilization on a chip.
- The method according to the invention enables fast chip surface functionalization with fluorescent probe beads and fast subsequent quantitative detection of analyte molecules.
- All ligand-based assays can be performed using the invention. In ligand-based assays at least one of the reaction partners is labeled with an entity whose quantity can be measured. Ligand-based assays include sandwich assays and competitive assays.
- The use of the method according to the invention in combination with on-chip bead-based affinity assays can be applied for various types of affinity assays, because many bead types with many probe molecule functionalizations are available today (versatility).

BRIEF DESCRIPTION OF THE DRAWINGS

Embodiments of the invention are described in greater detail hereinafter relative to the attached schematic drawings.

Figure 1        shows a flow diagram of the method according to the invention.

Figures 2-4     show three embodiments of the method according to the invention, (a) in flow diagrams, and (b) in schematic side views showing a microparticle and an analyte molecule.

Figure 5        schematically shows an experimental setup for performing the method according to the invention.

Figure 6        shows fluorescence images of fluorescent beads with a diameter (A) of 2 $\mu$m and (B) of 1 $\mu$m, respectively, immobilized on interdigitated electrodes.

Figure 7        shows in a diagram the manually counted number of beads versus their fluorescence signal.

Figure 8        shows images taken for various light wavelengths at various times during an experiment with two fluorescence-measurement steps.

Figure 9        shows in a diagram the fluorescence signal of bound streptavidin versus incubation time for various streptavidin concentrations.

Figure 10       shows in a diagram the fluorescence signal of bound streptavidin versus streptavidin concentration after 120 seconds of incubation time.

DESCRIPTION OF PREFERRED EMBODIMENTS

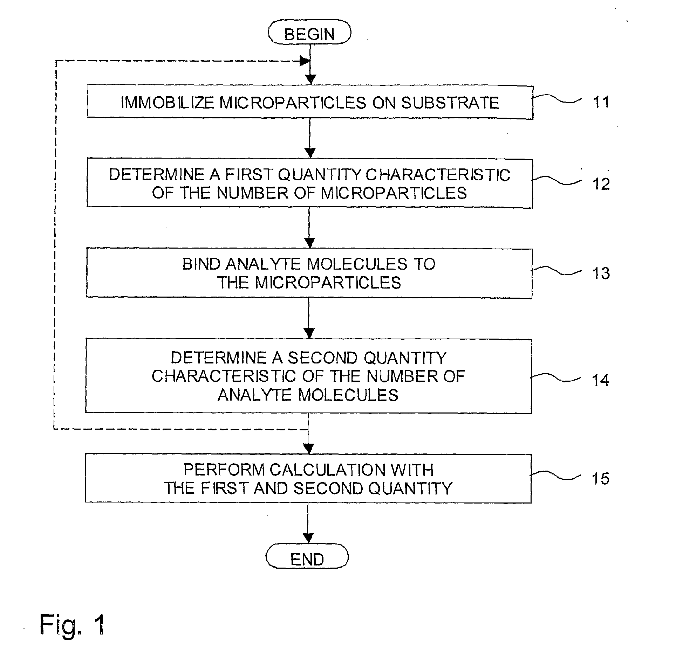

A flow diagram of the general method according to the invention is shown in **Figure 1.** The following discussion refers to an example in which fluorescence measurements are performed for determining quantities characteristic for the numbers of microparticles and of analyte molecules. This example shall not be limiting, since other ways of determining said quantities are possible and in places described in this document.

For realizing the invention, typically a microfluidic system, a microtiterplate or a chip with means for immobilizing beads in defined areas is required. Fluorescently dyed beads are functionalized with probe molecules and (reversibly or irreversibly) immobilized 11 in certain areas on the chip or in the microfluidic system. The immobilization 11 can be achieved, for example, by mechanical barriers, magnetic retention, dielectrophoretic retention or retention by dielectrophoresis-enhanced adhesion (cf. WO-2004/024333), retention in vortices generated by alternative-current electro-osmotic flow (AC EOF) and pressure-driven counterflow (cf. WO-02/092222), and/or other bead-immobilization mechanisms. Analyte molecules to be detected are labeled with a second fluorescent dye, and, if required, reference analyte molecules are labeled with a second fluorescent dye. The fluorescence of the bead dye should not excite the fluorescence of the analyte molecule dye, i.e., an appropriate combination of the two dyes must be chosen (e.g., fluorescein and Cy5, fluorescein and AlexaFluor 680, etc.).

A fluorescence measurement system able to detect the bead and analyte label fluorescence is provided. A first fluorescence measurement 12 determines the number of beads, which corresponds to the number of available binding sites for the following affinity assay. Alternatively, instead of measuring the integrated fluorescence signal of the beads, the bead number could also be determined using an image recognition software. However, the alternative approach will be very difficult for small bead sizes.

After immobilization 11, the beads are perfused with an analyte solution such that the analyte molecules are bound 13 to the beads. A second fluorescence signal characteristic of the number of analyte molecules bound to the bead surface is measured 14.

Steps 11-14 may be repeated once or several times. A sequence of repeated measurements may be necessary for calibration purposes.

Eventually, a calculation is performed 15 with the number of beads determined in the first fluorescence measurement 12 and the number of analyte molecules determined in the second fluorescence measurement 14. In the calculation 15, the second fluorescence measurement 14 is for instance corrected by the number of available binding sites, i.e., by the number of beads which is determined in the first fluorescence measurement 12. The correction 15 typically comprises a quotient calculation. Usually, especially for dynamic binding detection, a background-intensity correction is necessary. Also, the use of fluorescence standards is strongly recommended for both fluorescence measurements for further improvement of the measurement accuracy. A detailed example of a calculation 15 is given at the end of the description.

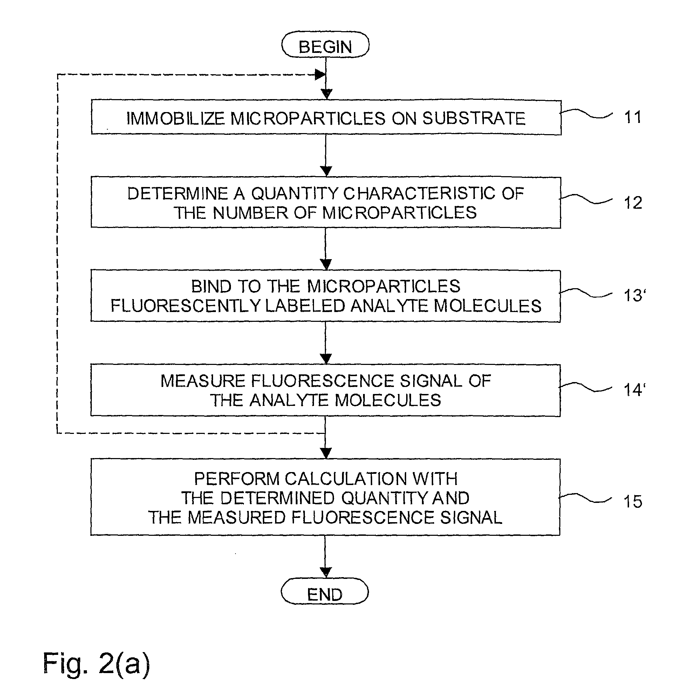

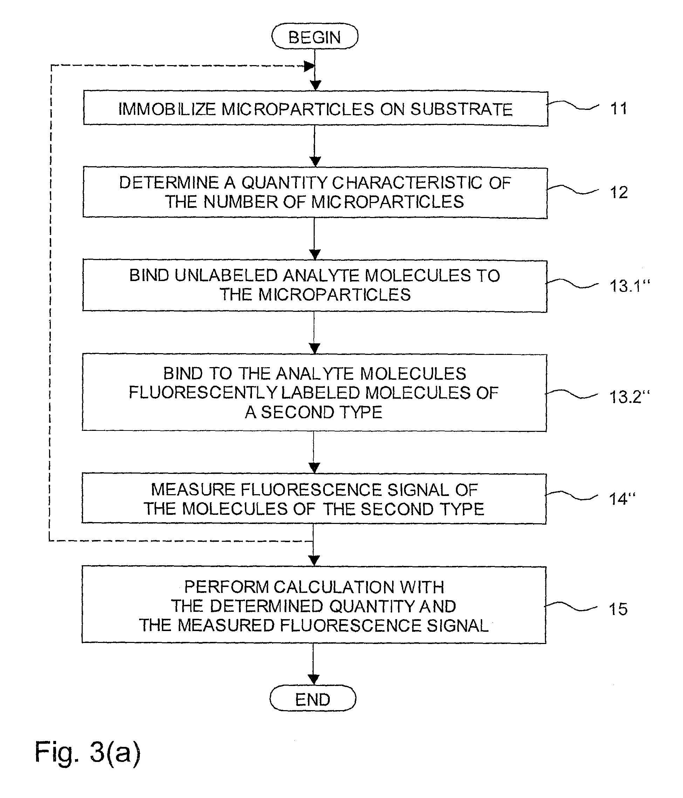

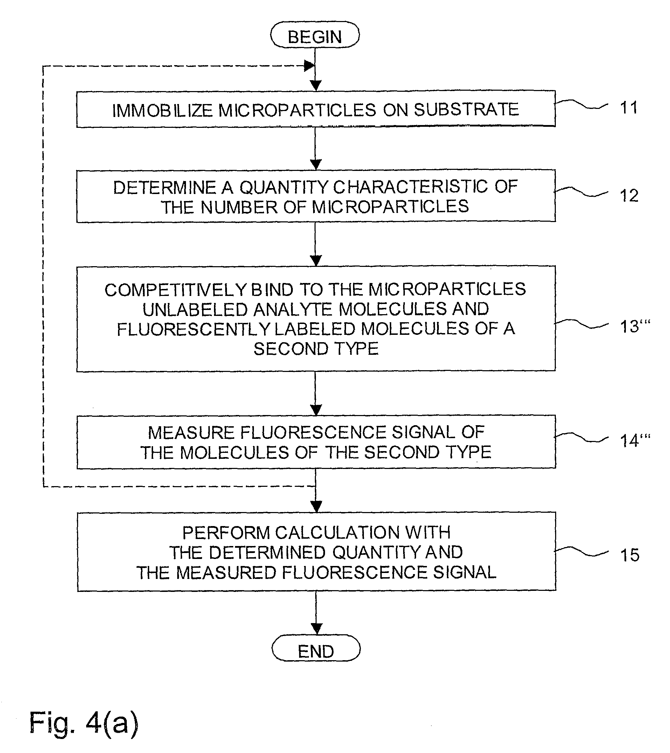

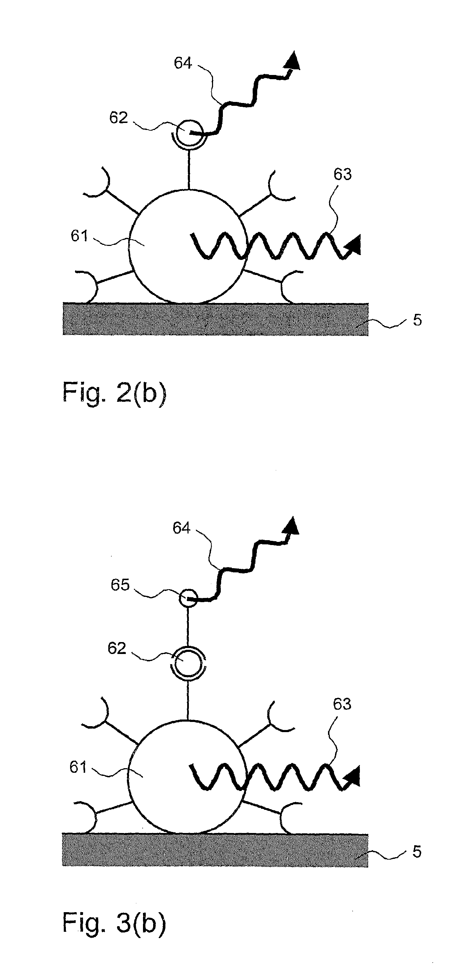

In **Figures 2-4,** three different ways of binding 13 analyte molecules 62 to microparticles 61 and of measuring a fluorescence signal 64 characteristic of the number of analyte molecules 62 are illustrated.

**Figure 2** shows an embodiment of the invention in which the analyte molecules 62 are able to emit fluorescent radiation 64. In other words: the analyte molecules 62 are fluorescent themselves or are fluorescently labeled. The analyte molecules 62 are bound to the microparticles 61, and their fluorescence signal 64 is measured 14'. The measured fluorescence signal 64 is proportional to the number of bound analyte molecules 62.

The embodiment of **Figure 3** is often called a "sandwich assay". According to this embodiment, unlabeled analyte molecules 62 are bound 13.1" to the microparticles 61. Subsequently, fluorescent molecules 65 are bound 13.2" to the analyte molecules 62. The fluorescence signal 64 of the fluorescent molecules 65 is measured 14". The measured fluorescence signal 64 is proportional to the number of bound analyte molecules 62.

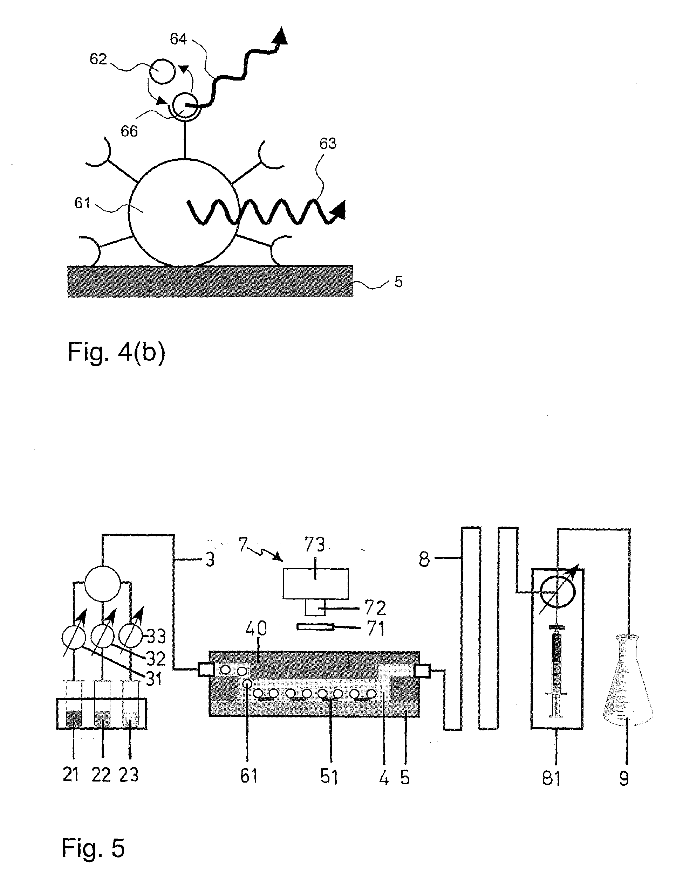

An embodiment called "competitive assay" is shown in **Figure 4.** Unlabeled analyte molecules 62 and fluorescent competitor molecules 66 are competitively bound 13''' to the microparticles. In this embodiment, a weak fluorescence signal 64 indicates a large number of bound analyte molecules 62, and vice versa.

**Figure 5** schematically shows a possible experimental setup for performing the method according to the invention. The setup comprises a plurality of reservoirs 21, 22, 23 containing media required for the measurement. In the exemplified embodiment of Fig. 5, a first reservoir 21 contains a bead suspension, a second reservoir 22 contains an analyte solution, and a third reservoir 23 contains a buffer solution. By means of appropriate valves 31, 32, 33, the media can be introduced into an inlet channel 3, separately or in mixture. The inlet channel 3 is connected to a fluidic chamber 4 formed, e.g., in a PMMA housing 40. Dimensions of the fluidic chamber 4 are, e.g., 10 mm in length, 4 mm in width and 30 $\mu$m in height. The fluidic chamber 4 is at least partly bounded by a substrate 5, on the surface of which functionalized beads 61 can be immobilized. The substrate 5 can be, e.g., a glass chip wich a plurality of platinum electrodes 51 on its surface. The platinum electrodes 51 are, e.g., 100 nm thin. The beads preferably have a diameter between 0.5 $\mu$m and 5 $\mu$m. There are provided means 7 for detecting fluorescence on the surface of the substrate 5, e.g., a microscope 72 with a camera 73. An appropriate spectral filter 71 or a plurality of exchangeable spectral filters for selecting a spectral range to be detected by the camera 73 can be arranged in the light path between the substrate 5 and the camera 73. An outlet channel 8 connects the fluidic chamber 4 with a drain container 9. A pump 81, which may be of any convenient form but is typically a syringe pump, is use to move the media through the fluidic chamber 4 and the channels 3, 8.

The fluorescence of the microparticles 61 is preferably measured 12 in a first spectral range that differs from and does not overlap with a second spectral range in which the fluorescence of the analyte molecules is measured 14. The light wavelengths of the first spectral range may be between approximately 450 nm and 600 nm, and the light wavelengths of the second spectral range may be between approximately 600 nm and 750 nm. In a first embodiment, the fluorescence in the first and the second spectral range is measured in two subsequent steps 12, 14, using a single photodetector 73 and two different spectral filters 71 for the two respective steps 12, 14. In a second, alternative embodiment, the fluorescence in the first and the second spectral range is measured in one step, using a single photodetector adapted for distinguishing between light within the first and the second spectral range, respectively, and preferably using a spectral filter that simultaneously transmits light wavelengths within the first and the second spectral range. In a third embodiment, the fluorescence in the first and the second spectral range is measured in one step, using means for separating light within the first spectral range from light within the second spectral range, and using two photodetectors for detecting the two respective light portions.

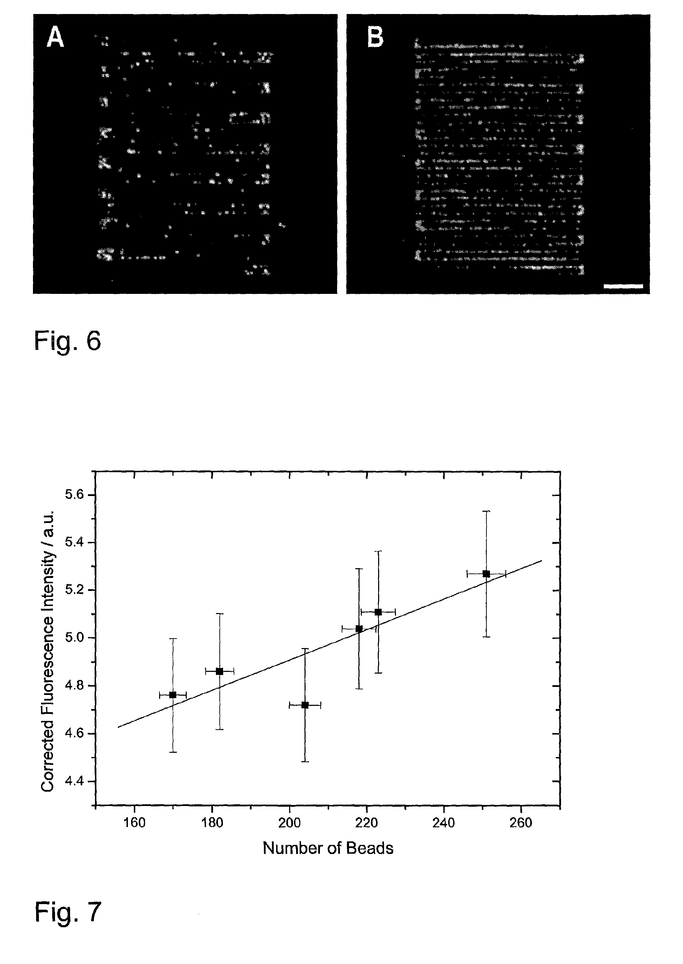

**Figure 6** shows fluorescence images of yellow-green biotin-coated beads immobilized on interdigitated electrodes. The beads had a diameter (A) of 2 $\mu$m and (B) of 1 $\mu$m, respecitvely. The 1 $\mu$m beads (Fig. 6B) adhere better and more homogeneously to the electrodes. The length of the scale bar in Fig. 6B is 50 $\mu$m.

The results of a quantitative bead-adhesion experiment are shown in the diagram of **Figure 7.** The experiment was performed with yellow-green fluorescent biotin-coated beads of 2 $\mu$m in diameter. The diagram shows the manually counted number of beads versus their fluorescence signal which was corrected by subtracting the background noise. The experiment demonstrates that the fluorescence signal is linearly proportional to the number of immobilized beads.

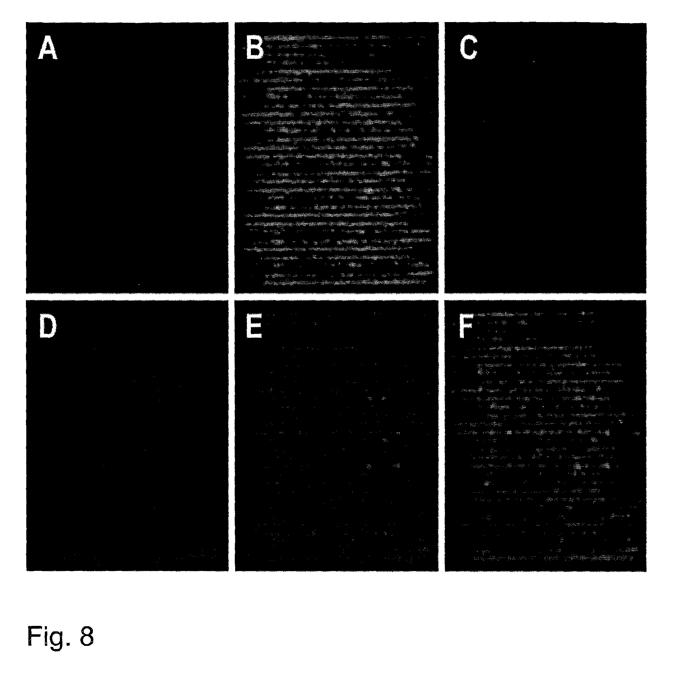

An experimental example for method according to the invention is discussed with reference to **Figures 8-10.** Biotin-coated yellow-green fluorescent beads of 1 $\mu$m in diameter were immobilized at the interdigitated electrode pads as described above. After flushing away excess beads, two fluorescence images were taken. The first image measured the yellow-green fluorescence of the beads at the electrode pads. The background noise without beads measured left and right of the electrode pad was subtracted from the measured intensity. The thus obtained signal was proportional the number of immobilized beads and hence to the number of available binding sites. The second image measured the red fluorescence intensity to verify that no red signal was present.

Then the dynamic binding detection of streptavidin labeled with a red fluorescence marker started. The beads were perfused with streptavidin solution at a pump rate of 0.2 $\mu$l/s. A red fluorescence picture integrating 50 frames on the camera chip (exposure time approximately 5 seconds) was taken every 15 seconds. The red fluorescence of the Alexa Fluor 680 dye was not visible to the human eye but to the camera. In order to measure only the intensity coming from streptavidin bound to the biotin bead surfaces, the background noise coming from the non-bound streptavidin solution was measured left and right of the electrode pad and subtracted from the intensity measured above the electrode pad. Finally, the streptavidin signal was corrected by a factor representing the number of available biotin beads or binding sites.

**Figure 8** shows images taken for various light wavelengths at various times during the experiment. **Figure 8A** is a yellow-green fluorescence image of the empty chip. **Figure 8B** is a yellow-green fluorescence image of immobilized yellow-green fluorescent beads (polystyrene, fluorescein-dyed) of 1 $\mu$m in diameter coated with biotin probe molecules. **Figure 8C** is a red fluorescence image of the situation of Fig. 8B; no red fluorescence signal is coming from the immobilized beads. **Figure 8D** is a red fluorescence image after perfusion with AlexaFluor-680 (red fluorescent dye) labeled streptavidin solution, incubation time 30 seconds. **Figure 8E** is a red fluorescence image: after incubation time of 60 seconds. **Figure 8F** is a red fluorescence image after incubation time of 120 seconds.

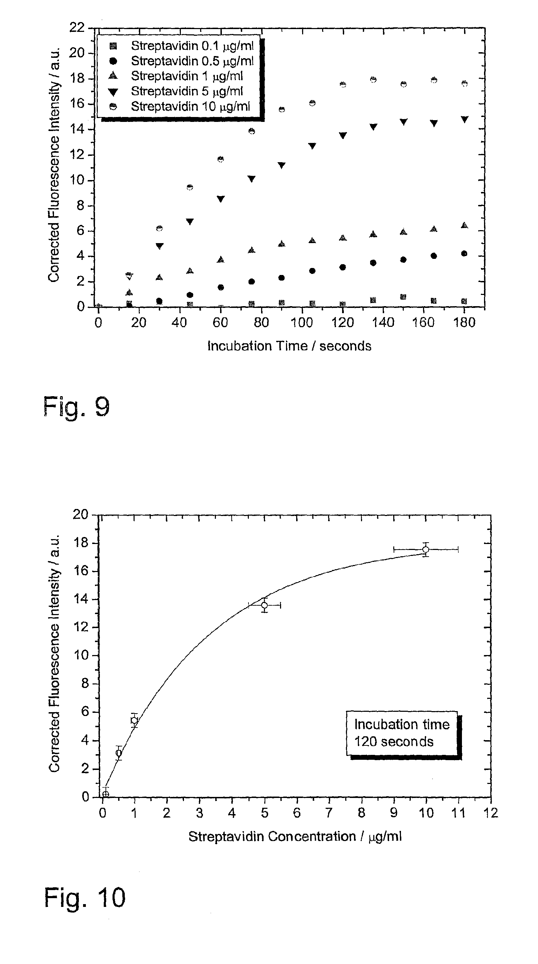

**Figure 9** shows dynamic streptavidin-biotin binding curves for various streptavidin concentrations. The diagram shows the fluorescence signal of bound streptavidin versus incubation time. The measured red-fluorescence intensity signal was corrected by the background intensity of unbound streptavidin still in solution and by the number of available biotin-coated beads.

**Figure 10** shows the fluorescence signal of bound streptavidin versus streptavidin concentration after 120 seconds of incubation time. The recommended dynamic range under the given assay conditions is between 0.1 and 10 $\mu$g/ml. The line curve is not a theoretical fit curve but emphasizes the saturation of available binding sites at higher concentrations.

In the following, one possible way for performing a calculation 15 (cf. Fig. 1) according to the invention is illustrated.

The quantities involved are:

| | |
|---|---|
| A | First quantity, characteristic of the number of beads, determined in the first fluorescence measurement 11; |
| a | Background noise during the first fluorescence measurement 11; |
| B | Second quantity, characteristic of the number of the analyte molecules, determined in the second fluorescence measurement 13; |
| b | Background noise during the second fluorescence measurement 13; |
| M | Number of bound analyte molecules; |
| N | Number of immobilized beads; |
| S | Number of available binding sites (binding capacity); |
| X | Fluorescence of a bead; |
| Y | Number of binding sites per bead, usually indicated by the bead provider; |
| Z | Flurescence of an analyte molecule. |
| (A - a) | is the fluorescence signal of the immobilized beads, and |
| (B - b) | is the fluorescence signal of the bound analyte molecules. |

Moreover, the following equations hold:

$$N = \frac{A - a}{X}$$

$$S = N \cdot Y$$

$$M = \frac{B - b}{Z}$$

The quantity M is influenced by a plurality of quantities, such as the perfusion time, the concentration of the analyte molecules in the analyte solution, the flow velocity of the analyte solution during dynamic binding, the reaction constant, the diffusion coefficient of the analyte molecules, the cross section of the fluidic chamber 4, and the number of binding sites available per area. The dynamic detection region of the analyte concentration and the detection limit can be modified by changing the flow velocity of the analyte solution during the dynamic binding process. If different bead charges have different binding capacities S, the binding capacities given by the bead manufacturer can be used to calculate corresponding correction factors. If all bead charges have the same binding capacity S, a capacity-dependent correction is not required.

In a first calculation step, a calibration curve of the kind as shown in Fig. 10 is determined. For determining the calibration curve, a plurality of calibration fluorescence measurements performed for different known analyte concentrations $c_1, c_2, ..., c_n$, is needed, namely:

$A_1, a_1; B_1, b_1$ for a first concentration $c_1$;
$A_2, a_2; B_2, b_2$ for a second concentration $c_2$;
$A_n, a_n; B_n, b_n$ for an $n^{th}$ concentration $c_n$.

One of the bead-fluorescence measurements, e.g., the pair $A_1, a_1$, is used as a reference for calculating correction coefficients $F_1, F_2, ... F_n$, according to the following formulas:

$$F_1 = \frac{A_1 - a_1}{A_1 - a_1} = 1$$

$$F_2 = \frac{A_2 - a_2}{A_1 - a_1}$$

$$F_n = \frac{A_n - a_n}{A_1 - a_1}$$

These correction coefficients $F_1, F_2, ... F_n$ are used for correcting the analyte-fluorescence measurements:

$\dfrac{(B_1 - b_1)}{F_1}$ is the corrected first measurement value,

$\dfrac{(B_2 - b_2)}{F_2}$ is the corrected second measurement value,

$\dfrac{(B_n - b_n)}{F_n}$ is the corrected $n^{th}$ measurement value.

The corrected analyte-fluorescence measurement values are plotted versus the corresponding concentrations $c_1, c_2, ..., c_n$, thus yielding a calibration curve as shown in Fig. 10.

In a second calculation step, an unknown analyte concentration $C_x$ is determined from measurement results $A_x, a_x; B_x, b_x$. For this purpose, a correction coefficient

$$F_x = \frac{A_x - a_x}{A_1 - a_1}$$

is calculated and used for correcting the analyte-fluorescence measurements to yield a corrected measurement value

$$\frac{(B_x - b_x)}{F_x} \quad .$$

From the calibration curve (cf. Fig. 10), the required concentration $c_x$ corresponding to the corrected value indicated above is determined. Of course, it is not necessary to physically plot the calibration curve; the calculation may be performed purely mathematically.

This invention is not limited to the preferred embodiments described above, to which variations and improvements may be made, without departing from the scope of protection of the present patent.

LIST OF REFERENCE SIGNS

11    Immobilization of microparticles on substrate
12    Determination of a first quantity characteristic of the number of microparticles
13    Binding analyte molecules to the microparticles
14    Determination of a second quantity characteristic of the number of the analyte molecules
15    Performing a calculation with the first and second quantity

21    Bead-suspension reservoir
22    Analyte-solution reservoir
23    Buffer-solution reservoir

3      Inlet channel
31-33  Valves

4    Fluidic chamber
40    Housing for fluidic chamber

5    Substrate
51    Electrodes on substrate

61    Microparticles/beads
62    Analyte molecules
63    Fluorescent signal from microparticle
64    Fluorescent signal from analyte molecule
65    Fluorescent molecule in sandwich assay
66    Fluorescent competitor molecule in competitive assay

7      Detection means
71    Spectral filter
72    Microscope
73    Camera

8      Outlet channel
81    Pump

9     Drain container

**Claims**

1.  A method for quantitative evaluation of bead-based affinity assays, comprising the steps of:

    immobilizing (11) microparticles (61) on a substrate (5); determining (12) a first quantity characteristic of the number of microparticles (61) immobilized on a certain area of the substrate (5);
    binding (13) analyte molecules (62) to the microparticles (61);
    determining (14) a second quantity characteristic of the number of analyte molecules (62) bound in said area of the substrate (5); and
    performing (15) a calculation with the first and the second quantity.

2.  The method according to claim 1, wherein the second quantity is selected from the group consisting of a fluorescence signal (64), a signal generated by colloidal gold labels, a signal generated by enzymatic activity, an electro-chemo-luminescence signal and an enzyme-linked immuno-sorbent assay signal.

3.  The method according to claim 1 or 2, wherein the affinity assay is a ligand assay such as a sandwich assay or a competitive assay.

4.  The method according to any of the preceding claims, wherein the first quantity is determined (12) by providing fluorescent microparticles (61) and measuring a fluorescence signal (63) emitted by the microparticles (61) in said area of the substrate (5).

5.  The method according to claims 2 and 4, wherein the first quantity is determined (12) by measuring a first fluorescence signal (63) in a first spectral range and the second quantity is determined (14) by measuring a second fluorescence signal (64) in a second spectral range, and the first spectral range differs from and does not overlap with the second spectral range.

6.  The method according to claim 5, wherein the light wavelengths of the first spectral range are between approximately 450 nm and 600 nm and the light wavelengths of the second spectral range are between approximately 600 nm and 750 nm.

7.  The method according to claim 5 or 6, wherein the fluorescence signals (63, 64) in the first and the second spectral ranges are measured in two subsequent steps (12, 14), using a single photodetector (73) and two different spectral filters (71) for the two respective steps (12, 14).

8.  The method according to claim 5 or 6, wherein the fluorescence signals (63, 64) in the first and the second spectral ranges are measured in one step, using a single photodetector (73) adapted for distinguishing between light within the first and the second spectral range, respectively, and preferably using a spectral filter (71) that simultaneously transmits light wavelengths within the first and the second spectral range.

9.  The method according to claim 5 or 6, wherein the fluorescence signals (63, 64) in the first and the second spectral ranges are measured in one step, using means for separating light (63) within the first spectral range from light (64) within the second spectral range, and using two photodetectors for detecting the two respective light portions.

10. The method according to any of the preceding claims, wherein analyte molecules (62) are bound (13') to the microparticles (61), and a signal (64) emitted by the analyte molecules (62) is measured (14') for determining the second quantity.

11. The method according to any of the claims 1-9, wherein unlabeled analyte molecules (62) are bound (13.1") to the microparticles (61), molecules (65) of a second type are bound (13.2'') to the analyte molecules (62), and a signal (64) emitted by the molecules (65) of the second type is measured (14'') for determining the second quantity.

12. The method according to any of the claims 1-9, wherein unlabeled analyte molecules (62) and molecules (66) of a second type are competitively bound (13''') to the microparticles (61), and a signal (64) emitted by the molecules (66) of the second type is measured (14''') for determining the second quantity.

13. The method according to any of the preceding claims, wherein the microparticles (61) are beads, preferably with a diameter between 0.5 $\mu$m and 5 $\mu$m.

14. The method according to any of the preceding claims, wherein the microparticles (61) are immobilized (11) on the substrate (5) by a method selected from the group consisting of mechanical barriers, magnetic retention, dielectrophoretic retention, retention by dielectrophoresis-enhanced adhesion, retention in vortices generated by alternative-current electro-osmotic flow, and pressure-driven counterflow.

15. The method according to any of the preceding claims, wherein the calculation performed (15) with the first quantity and the second quantity comprises the calculation of a quotient of the second quantity and the first quantity or vice versa.

16. The method according to claim 15, wherein prior to calculating the quotient, a background noise is determined and subtracted from the first and/or the second quantity.

17. The method according to any of the preceding claims, wherein the calculation (15) performed with the first quantity and the second quantity further takes into account a number of binding sites per microparticle (61).

Fig. 1

BEGIN

IMMOBILIZE MICROPARTICLES ON SUBSTRATE — 11

DETERMINE A QUANTITY CHARACTERISTIC OF
THE NUMBER OF MICROPARTICLES — 12

BIND TO THE MICROPARTICLES
FLUORESCENTLY LABELED ANALYTE MOLECULES — 13'

MEASURE FLUORESCENCE SIGNAL OF
THE ANALYTE MOLECULES — 14'

PERFORM CALCULATION WITH
THE DETERMINED QUANTITY AND
THE MEASURED FLUORESCENCE SIGNAL — 15

END

## Fig. 2(a)

BEGIN

IMMOBILIZE MICROPARTICLES ON SUBSTRATE — 11

DETERMINE A QUANTITY CHARACTERISTIC OF
THE NUMBER OF MICROPARTICLES — 12

BIND UNLABELED ANALYTE MOLECULES TO
THE MICROPARTICLES — 13.1"

BIND TO THE ANALYTE MOLECULES
FLUORESCENTLY LABELED MOLECULES OF
A SECOND TYPE — 13.2"

MEASURE FLUORESCENCE SIGNAL OF
THE MOLECULES OF THE SECOND TYPE — 14"

PERFORM CALCULATION WITH
THE DETERMINED QUANTITY AND
THE MEASURED FLUORESCENCE SIGNAL — 15

END

Fig. 3(a)

```
                    ( BEGIN )
                         |
  +----------------------|
  |                      v
  |   +-------------------------------------+
  |   |  IMMOBILIZE MICROPARTICLES ON SUBSTRATE  |------  11
  |   +-------------------------------------+
  |                      |
  |                      v
  |   +-------------------------------------+
  |   |  DETERMINE A QUANTITY CHARACTERISTIC OF   |------  12
  |   |     THE NUMBER OF MICROPARTICLES          |
  |   +-------------------------------------+
  |                      |
  |                      v
  |   +-------------------------------------+
  |   |  COMPETITIVELY BIND TO THE MICROPARTICLES  |
  |   |  UNLABELED ANALYTE MOLECULES AND           |------  13'''
  |   |  FLUORESCENTLY LABELED MOLECULES OF A      |
  |   |  SECOND TYPE                               |
  |   +-------------------------------------+
  |                      |
  |                      v
  |   +-------------------------------------+
  |   |  MEASURE FLUORESCENCE SIGNAL OF           |------  14'''
  |   |  THE MOLECULES OF THE SECOND TYPE         |
  |   +-------------------------------------+
  +----------------------|
                         v
      +-------------------------------------+
      |  PERFORM CALCULATION WITH                 |
      |  THE DETERMINED QUANTITY AND              |------  15
      |  THE MEASURED FLUORESCENCE SIGNAL         |
      +-------------------------------------+
                         |
                         v
                    (  END  )
```

# Fig. 4(a)

Fig. 2(b)

Fig. 3(b)

Fig. 4(b)

Fig. 5

Fig. 6

Fig. 7

Fig. 8

EP 1 653 232 A1

Fig. 9

Fig. 10

17

**European Patent Office**

**EUROPEAN SEARCH REPORT**

Application Number

EP 04 40 5661

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | US 2004/110222 A1 (SHIMAMOTO NOBUO ET AL) 10 June 2004 (2004-06-10) * the whole document * ----- | 1-17 | G01N33/543 |
| X | US 2003/170447 A1 (TANAAMI TAKEO ET AL) 11 September 2003 (2003-09-11) * abstract * * page 1, right-hand column, paragraph 10 * * page 2, left-hand column, paragraphs 1,2 * ----- | 1-4 | |
| X | WO 02/39083 A (PEREZ LUNA VICTOR H ; SCIENCE & TECHNOLOGY CORP UNM (US); BURANDA TION) 16 May 2002 (2002-05-16) * abstract * * page 7, paragraph 3 * * page 16, last paragraph * * page 17, paragraph FIRST * * page 25, last paragraph * * page 39, paragraphs 2,3; figures 4,6 * ----- | 1,3,4, 10-14 | |
| A | US 2002/098588 A1 (FRIEDMAN ALEX ET AL) 25 July 2002 (2002-07-25) * the whole document * ----- | 1-17 | TECHNICAL FIELDS SEARCHED (Int.Cl.7) G01N |

The present search report has been drawn up for all claims

| Place of search | Date of completion of the search | Examiner |
|---|---|---|
| Munich | 28 January 2005 | Weijland, A |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or after the filing date
D : document cited in the application
L : document cited for other reasons

........................................................................

& : member of the same patent family, corresponding document

**ANNEX TO THE EUROPEAN SEARCH REPORT
ON EUROPEAN PATENT APPLICATION NO.**                    EP 04 40 5661

This annex lists the patent family members relating to the patent documents cited in  the above-mentioned European search report.
The members are as contained in the European Patent Office EDP file on
The European Patent Office is in no way liable for these particulars which are merely  given for the purpose of information.

28-01-2005

| Patent document cited in search report | | Publication date | Patent family member(s) | | Publication date |
|---|---|---|---|---|---|
| US 2004110222 | A1 | 10-06-2004 | JP | 2004184312 A | 02-07-2004 |
| US 2003170447 | A1 | 11-09-2003 | JP | 2003262639 A | 19-09-2003 |
| WO 0239083 | A | 16-05-2002 | AU | 3041902 A | 21-05-2002 |
| | | | WO | 0239083 A2 | 16-05-2002 |
| | | | US | 2002081617 A1 | 27-06-2002 |
| US 2002098588 | A1 | 25-07-2002 | NONE | | |

EPO FORM P0459

For more details about this annex : see Official Journal of the European Patent Office, No. 12/82